# EUROPÄISCHE PATENTSCHRIFT

(11) **EP 0 970 040 B1**
(45) Veröffentlichungstag und Bekanntmachung des Hinweises auf die Patenterteilung: **12.06.2002**
(21) Anmeldenummer: 98912399.7
(22) Anmeldetag: 26.02.1998
(51) Int. Cl.: C07C 209/10, C07C 211/48, C07D 295/06, C07D 295/12

(54) **VERFAHREN ZUR HERSTELLUNG VON N-CARBOXYALKYL-3-FLUOR-4-DIALKYLAMINOANILINEN**
METHOD FOR PRODUCING N-CARBOXYALKYL-3-FLUORO-4-DIALKYLAMINONILINES
PROCEDE DE PREPARATION DE N-CARBOXYALKYLE-3-FLUORO-4-DIALKYLAMINOANILINES

(30) Priorität: 07.03.1997 DE 19709439; 07.03.1997 DE 19709441; 07.03.1997 DE 19709442
(43) Veröffentlichungstag der Anmeldung: 12.01.2000
(73) Patentinhaber: Clariant GmbH, 65929 Frankfurt am Main (DE)
(72) Erfinder: SCHACH, Thomas, IN-Mumbai 400034 (IN); PAPENFUHS, Theodor, D-60433 Frankfurt (DE)
(86) Internationale Anmeldenummer: EP9801085
(87) Internationale Veröffentlichungsnummer: WO9840348

(56) Entgegenhaltungen:
- WO-A-95/25106
- STEVEN J. BRICKNER ET AL.: "Synthesis and Antibacterial Activity of U-100592 and U-100766, Two Oxazolidinone Antibacterial Agents for the Potential Treatment of Multidrug-Resistant Gram-Positive Bacterial Infections" JOURNAL OF MEDICINAL CHEMISTRY, Bd. 39, Nr. 3, 2.Februar 1996, WASHINGTON US, Seiten 673-679, XP002070209
- REN BEUGELMANS ET AL.: "Synthèse d'hétérocycles à 5 et 6 chaînons par une stratégie combinant des réactions SNAr et SRN1 " BULLETIN DE LA SOCIETE CHIMIQUE DE FRANCE., Bd. 132, Nr. 3, 1995, PARIS FR, Seiten 306-313, XP002070210
- VIOLETTA CECCHETTI ET AL.: "1,4-Benzothiazine-2-carboxylic Acid 1-Oxides as Analogues of Antibacterial Quinolones [1]" JOURNAL OF HETEROCYCLIC CHEMISTRY., Bd. 29, Nr. 2, 1992, PROVO US, Seiten 375-382, XP002070211

## Beschreibung

Die vorliegende Erfindung betrifft ein gegenüber dem Stand der Technik verbessertes Verfahren zur Herstellung von N-Carboxyalkyl-3-fluor-4-dialkylaminoanilinen, sowie die Verbindungen 2-Chlor-5-fluor-4-(diethylamino, morpholino, piperidino oder piperazino)nitrobenzol und 2-Chlor-5-fluor-4-(morpholino oder piperazino)anilin.

N-Carboxyalkyl-3-fluor-4-dialkylaminoaniline spielen als Zwischenprodukte bei der Herstellung von Pharmazeutika (WO-95/25106) eine bedeutende Rolle.

Wie aus der WO 95/25106 hervorgeht, dient N-Carboxybenzyl-3-fluor-4-piperidinoanilin als Vorprodukt zur Herstellung Oxazolidinonderivaten und diese Derivate enthaltenden pharmazeutischen Zusammensetzungen.

Zur Herstellung des N-Carboxybenzyl-3-fluor-4-piperidinoanilins (Beispiel 1 der WO 95/25106) setzt man zunächst in einem ersten Schritt 3,4-Difluornitrobenzol in Anwesenheit von Diisopropylethylamin mit Piperidin in Ethylacetat um, fügt zu der Reaktionslösung Wasser zu, trennt die Ethylacetatphase ab, wäscht diese mit Wasser und Salzlauge und trocknet über wasserfreiem Natriumsulfat. Anschließend dampft man das Lösungsmittel ab und erhält die Nitroverbindung (3-Fluor-4-piperidinonitrobenzol). Man löst die Nitroverbindung in Ethylacetat und hydriert in Gegenwart eines Palladiumkatalysators, filtriert den Katalysator ab, dampft unter Vakuum ein und erhält das entsprechende Amin (3-Fluor-4-piperidino-anilin). In einem dritten Schritt läßt man das in Tetrahydrofuran gelöste Amin mit Natriumhydrogencarbonat und Chlorameisensäureester reagieren und fügt nach Abschluß der Reaktion Wasser zu, trennt die Tetrahydrofuranlösung ab, wäscht sie mit Wasser und Salzlauge und trocknet über wasserfreiem Natriumsulfat. Nach Abdampfen des Lösungsmittels wird das Produkt mittels Säulenchromatographie gereinigt.

Die vorstehend beschriebene Synthese macht sich den Umstand zunutze, daß das in 4-Stellung stehende Fluor gegen einen Piperidinrest ausgetauscht wird. Es ist allerdings darauf aufmerksam zu machen, daß in dem als Ausgangsstoff verwendeten 3,4-Difluornitrobenzol nur eine Position für die Austauschreaktion in Frage kommt, nämlich die durch die Nitrogruppe aktivierte para-Stellung. Da sich in dem 3,4-Difluornitrobenzol keine weiteren, ebenfalls zum Austausch befähigten in ortho-Stellung zu der Nitrogruppe stehenden Gruppen befinden, verläuft der Austausch des in 4-Stellung befindlichen Fluors glatt und ohne jede Komplikation.

Eine Nitrogruppe führt bekanntermaßen zu einer Aktivierung von Halogensubstituenten sowohl in der ortho-Stellung als auch in der para-Stellung. Setzt man beispielsweise 2-Chlornitrobenzol mit Anilin bei 175 bis 205°C um, so erhält man 2-Nitrodiphenylamin in quantitativer Ausbeute. Hingegen reagiert 4-Chlornitrobenzol mit Anilin bei 175 bis 205°C überhaupt nicht. (Houben-Weyl, Methoden der Organischen Chemie, IV. Auflage, Band XI/1, Seite 63 und 64). Dieses Verhalten beweist, daß die Nitrogruppe das in ortho-Stellung stehende Chlor in sehr hohem Maße aktiviert, während seine aktivierende Wirkung auf das in para-Stellung stehende Chlor nicht mehr ausreicht, die Austauschreaktion zu ermöglichen.

Das in der WO 95/25106 beschriebene Verfahren zur Herstellung von N-Carboxybenzyl-3-fluor-4-piperidinoanilin weist mehrere Nachteile auf. Zum einen kann man lediglich ein einziges Edukt, nämlich 3,4-Difluornitrobenzol, verwenden und zum anderen handelt es sich bei dem 3,4-Difluornitrobenzol um ein recht kostspieliges Produkt, das sich nur über eine sehr aufwendige, mehrstufige Synthese herstellen läßt. Weitere Nachteile bestehen darin, daß das Verfahren sehr viele Einzelschritte erfordert und ein jedes Zwischenprodukt isoliert wird. Darüber hinaus erfordern die einzelnen Reaktionsschritte einen nicht unerheblichen Zeitaufwand, der für die erste Stufe 2 Tage und für die beiden anderen Stufen jeweils 14 Stunden beträgt.

Im Hinblick auf die vorangegangenen Darlegungen, besteht ein Bedarf, ein Verfahren zur Herstellung von N-Carboxyalkyl-3-fluor-4-dialkylaminonanilinen bereitzustellen, das die genannten Nachteile vermeidet und sich mit einem vertretbarem Arbeits- und Zeitaufwand realisieren läßt. Darüberhinaus soll sich dieses Verfahren nicht auf die Herstellung von N-Carboxyalkyl-3-fluor-4-piperidinoanilinen beschränken, sondern weitere Verbindungen aus dieser Stoffgruppe zugänglich machen.

Gelöst wird diese Aufgabe durch ein Verfahren zur Herstellung von N-Carboxyalkyl-3-fluor-4-dialkylaminoanilinen. Es ist dadurch gekennzeichnet, daß man in einem ersten Schritt ein ortho-Nitrochlorbenzol der Formel (1) 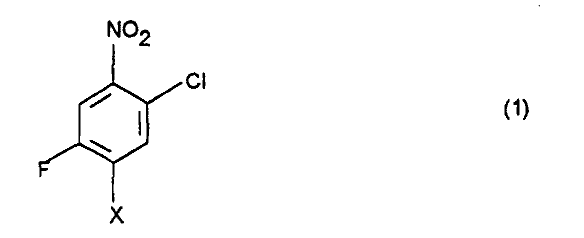 worin X für Cl oder F steht, mit einem sekundären Amin der Formel (2) HNR¹R², worin R¹ und R² unabhängig voneinander gleich oder verschieden sind und für einen Alkylrest mit 1 bis 10 Kohlenstoffatomen stehen oder zusammen mit dem N-Atom, an dem sie stehen, einen Ring mit 3 bis 7, insbesondere 5 bis 7 Gliedern bilden, in Gegenwart einer Base in Anwesenheit oder Abwesenheit eines Lösungsmittels bei -10 bis 120°C umsetzt, in einem zweiten Schritt das 2-Chlor-4-dialkylamino-5-fluornitrobenzol in Gegenwart einer Base und eines Edelmetallkatalysators mit Wasserstoff bei 30 bis 150°C und 1 bis 100 bar umsetzt, in einem dritten Schritt aus dem Reaktionsgemisch das 3-Fluor-4-dialkylaminoanilin mit einer wäßrigen Lösung einer Säure als in Wasser gelöstes Salz extrahiert, die wäßrige Phase abtrennt und das in Wasser gelöste Salz des 3-Fluor-4-dialkylaminoanilins in Gegenwart einer basischen Verbindung mit einem Chlorameisensäureester der Formel (3) CICO₂R³, worin R³ für einen Alkylrest mit 1 bis 10 Kohlenstoffatomen oder Aralkylrest mit 7 bis 20 Kohlenstoffatomen steht, bei 0 bis 100°C umsetzt.

Im Hinblick auf die vorangegangenen Ausführungen bezüglich der Umsetzung von 2-Chlomitrobenzol und 4-Chlornitrobenzol mit Anilin, die beweisen, daß der in para-Stellung zur Nitrogruppe stehende Chlorsubstituent keine für einen Austausch ausreichende Aktivierung besitzt, ist es als sehr überraschend anzusehen, daß in dem ortho-Nitrochlorbenzol der Formel (I) trotz der Anwesenheit eines durch die Nitrogruppe stark aktivierten ortho-Chlorsubstituenten ein Austausch des in para-Stellung zur Nitrogruppe stehenden Substituenten X erfolgt. Aufgrund der vorangegangenen Darlegungen bezüglich der unterschiedlichen Aktivierung wäre zu erwarten gewesen, daß lediglich ein Austausch des in ortho-Stellung stehenden Chlors erfolgen würde und zugleich ein Austausch des in para-Stellung stehenden Substituenten X unterbleiben würde. Es war nicht zu erwarten, daß für den Fall, daß X für Cl steht, überhaupt ein Austausch und erst recht nicht ein Austausch in nennenswertem Umfang stattfinden würde. Ferner war nicht zu erwarten, daß für den Fall, daß X für F steht, die Umsetzung mit hoher Selektivität und unter Austausch des in 4-Stellung stehenden Fluors erfolgt, während das in ortho-Stellung zur Nitrogruppe stehende Chlor im Molekül verbleibt.

Ein Vorteil des erfindungsgemäßen Verfahrens besteht darin, daß man von zwei verschiedenen Edukten, nämlich 2,4-Dichlor-5-fluornitrobenzol und 2-Chlor-4,5-difluomitrobenzol, ausgehen kann und zudem diese beiden Ausgangsstoffe wesentlich leichter als 3,4-Difluomitrobenzol zugänglich sind. Von Vorteil ist ferner, daß man bei Durchführung des erfindungsgemäßen Verfahrens nicht in jedem Fall darauf angewiesen ist, ein jedes Zwischenprodukt zu isolieren, bevor man es weiterverarbeitet. Man kann zwar die entsprechenden Zwischenprodukte isolieren, um sie anschließend weiterzuverarbeiten. Man kann aber auch vorteilhafterweise auf eine aufwendige Zwischenisolierung verzichten und das jeweils anfallende Reaktionsgemisch vor seiner Weiterverarbeitung einfachen Trennoperationen (Filtration, Extraktion) unterziehen und anschließend unmittelbar weiterverarbeiten.

Das erfindungsgemäße Verfahren zur Herstellung von N-Carboxyalkyl-3-fluor-4-dialkylaminoanilinen ist durch das nachfolgende Reaktionsschema in vereinfachter Form wiedergegeben.

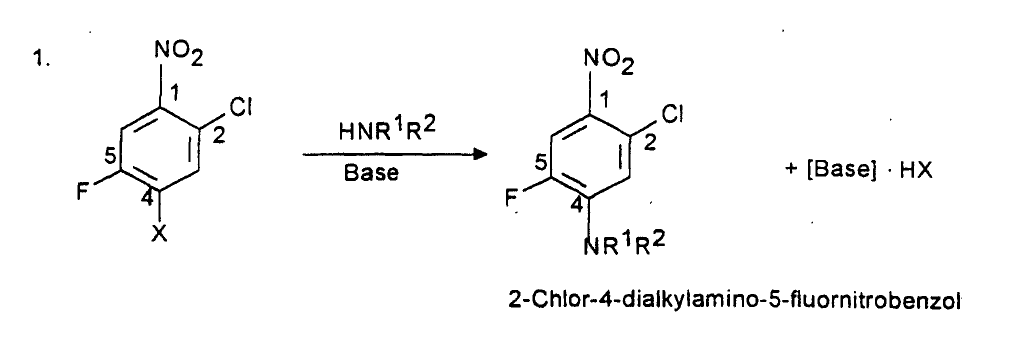

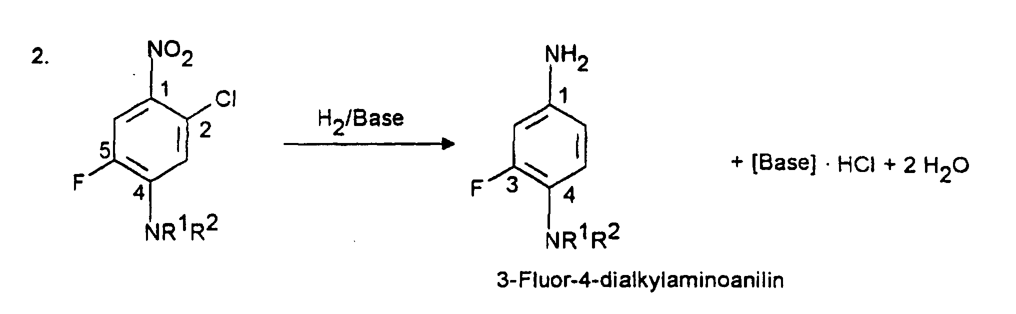

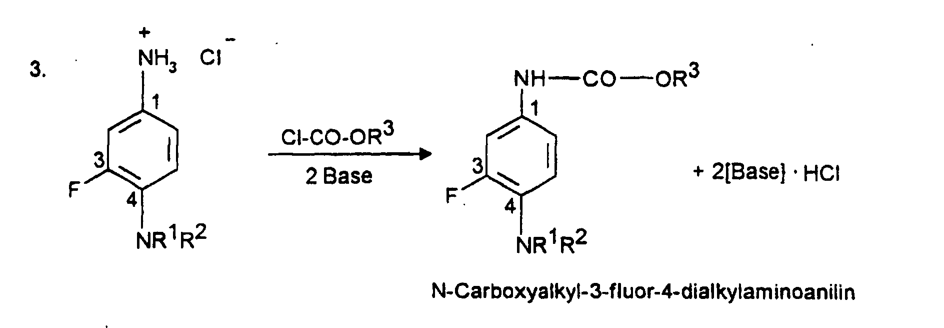

Wie zuvor bereits erwähnt, wird in einem ersten Schritt der im ortho-Nitrochlorbenzol der Formel (1) in para-Stellung zur Nitrogruppe stehende Substituent X durch Umsetzung mit dem sekundären Amin der Formel (II) in Gegenwart einer Base in Anwesenheit oder Abwesenheit eines Lösungsmittels gegen einen Aminrest -NR¹R² ausgetauscht.

Mit gutem Erfolg kann man ein ortho-Nitrochlorbenzol der Formel (1), worin X für F steht, einsetzen.

Man setzt als sekundäres Amin der Formel (2) Dimethylamin, Diethylamin, Di-n-propylamin, Di-i-propylamin, Di-n-butylamin, Di-i-butylamin, Piperidin, Morpholin oder Piperazin, insbesondere Piperidin, Morpholin oder Piperazin, bevorzugt Morpholin oder Piperazin ein.

Piperidin, Morpholin und Piperazin sind Beispiele für sekundäre Amine der Formel (2), worin R¹ und R² zusammen mit dem N-Atom, an dem sie stehen, einen Ring bilden. Dieser Ring weist jeweils 6 Glieder auf.

Man setzt in den ersten Schritt als Base, wie zuvor bereits erwähnt, das sekundäre Amin der Formel (2) oder ein tertiäres Amin, insbesondere das sekundäre Amin der Formel (2) oder ein Trialkylamin, dessen Reste gleich oder verschieden sind und 1 bis 25, insbesondere 6 bis 25, bevorzugt 8 bis 12 Kohienstoffatome je Alkylrest aufweisen, bevorzugt N-Triisooctylamin, Trialkyl(C₈/C₁₀)amine, Tri-N-dodecylamin oder ein Gemisch derselben ein.

Als Base, die wasserunlösliche, in organischer Phase lösliche Hydrochloride bilden, eignen sich insbesondere Trialkylamine mit 7 bis 12 Kohlenstoffatomen je Alkylrest, wobei die Alkylreste gleich oder verschieden sein können, vorzugsweise Triisooctylamin, Trialkyl(C₈/C₁₀)amine (ein Gemisch von Trialkylaminen mit 8 bis 10 Kohlenstoffatomen je Alkylrest, beispielsweise Hostarex A 327, ein Handelsprodukt der Hoechst AG), Tri-(N-dodecyl)amin. Diese Trialkylamine eignen sich besonders als Base für den ersten Schritt der Reaktionsfolge.

Man setzt im ersten Schritt der Reaktionsfolge die Base in einer Menge von 50 bis 500, insbesondere 100 bis 250, bevorzugt 100 bis 130 Mol%, bezogen auf Äquivalent abzuspaltendes Cl oder F ein.

Aufgabe der Base ist es, den bei der Umsetzung sich bildenden Fluorwasserstoff oder Chlorwasserstoff zu binden. Als Base kann das sekundäre Amin, das in diesem Fall in entsprechendem Überschuß einzusetzen ist, Anwendung finden. Es kann aber auch - wir zuvor angegeben - ein tertiäres Amin, insbesondere ein Trialkylamin als Base eingesetzt werden. Nach Beendigung der Umsetzung trennt man die aus der Base gebildeten Salze, beispielsweise durch Filtration oder Dekantieren ab, oder behandelt das Reaktionsprodukt mit wäßriger Lauge, beispielsweise wäßriger NaOH und/oder KOH Lösung, setzt das als Base verwendete sekundäre oder tertiäre Amin frei und trennt die wäßrige NaF und/oder KF respektive NaCI und/oder KCI enthaltende Lösung ab.

Diese Variante ist besonders vorteilhaft, da sie zum einen sehr einfach durchzuführen ist - eine Phasentrennung erfordert weniger Arbeitsaufwand als eine Filtration - und zum anderen das als Base verwendete sekundäre oder tertiäre Amin im Reaktionsprodukt verbleibt und mit diesem in die nächste Stufe, die ebenfalls in Gegenwart einer Base durchgeführt wird, gelangt.

Aus dem dabei anfallenden Reaktionsgemisch kann das 2-Chlor-4-dialkylamino-5-fluomitrobenzol isoliert und anschließend in isolierter Form weiterverarbeitet werden. Es ist allerdings auch möglich, das anfallende Reaktionsgemisch unmittelbar weiterzuverarbeiten.

Nach einer weiteren Variante setzt man in den ersten Schritt, bezogen auf das ortho-Nitrochlorbenzol der Formel (1) ≥ 200 Mol% tertiäres Amin als Base ein, so kann man das anfallende Reaktionsgemisch direkt, ohne Behandlung mit wäßriger Lauge, in den zweiten Schritt einsetzen und hydrieren.

Setzt man 2,4-Dichlor-5-fluornitrobenzol (X = Cl) als Ausgangsstoff ein, so empfiehlt es sich, das gewünschte 2-Chlor-4-dialkylamino-5-fluomitrobenzol, beispielsweise durch Kristallisation, abzutrennen und als gereinigtes Produkt weiterzuverarbeiten.

Mit besonders gutem Erfolg kann man in das erfindungsgemäße Verfahren - wie zuvor erwähnt - ein ortho-Nitrochlorbenzol der Formel (1), worin für X = F steht, einsetzen.

Setzt man nämlich 2-Chlor-4,5-difluomitrobenzol (X = F) als Ausgangsstoff ein, so kann man auf eine Isolierung des 2-Chlor-4-dialkylamino-5-fluornitrobenzols verzichten und das im ersten Schritt anfallende Reaktionsgemisch nach Behandlung mit wäßriger Lauge unmittelbar weiterverarbeiten. Diese Variante des erfindungsgemäßen Verfahren ist besonders vorteilhaft, da sie sich besonders einfach durchführen läßt. Setzt man in den ersten Schritt bezogen auf das 2-Chlor-4,5-difluornitrobenzol ≥ 200 Mol% tertiäres Amin als Base ein, so kann man das anfallende Reaktionsgemisch direkt, ohne Behandlung mit wäßriger Lauge, in den zweiten Schritt einsetzen und hydrieren.

Setzt man ein ortho-Nitrochlorbenzol der Formel (1), worin X für F steht, also 2-Chlor-4,5-difluomitrobenzol ein, so kann man die vorstehend genannten Lösungsmittel, insbesondere Toluol, ortho-Xylol, meta-Xylol, para-Xylol, ein Gemisch isomerer Xylole, Essigsäure(C₁-C₄)alkylester oder ein Gemisch dieser Lösungsmittel als Lösungsmittel einsetzen.

Setzt man hingegen das ortho-Nitrochlorbenzol der Formel (1), worin X für Cl steht, also 2,4-Dichlor-5-fluor-nitrobenzol ein, so empfiehlt es sich, ein dipolar aprotisches Lösungsmittel, z.B. Dimethylformamid, Dimethylacetamid, 1,3-Dimethylimidazolidin-2-on oder ein Gemisch derselben als Lösungsmittel zu verwenden.

Wie bereits erwähnt, läßt sich das Verfahren in Anwesenheit oder Abwesenheit eines Lösungsmittels durchführen.

In einer Vielzahl von Fällen wird man sowohl im ersten Schritt als auch im zweiten Schritt der Verwendung eines Lösungsmittels den Vorzug geben. Zur Durchführung des Verfahrens eignet sich eine Vielzahl verschiedener Lösungsmittel. Hierunter fallen unpolare Lösungsmittel, aprotische Lösungsmittel, dipolar aprotische Lösungsmittel und polar aprotische Lösungsmittel.

Ohne Anspruch auf Vollständigkeit zu erheben, sei erwähnt, daß man als Lösungsmittel einen aliphatischen Kohlenwasserstoff mit 5 bis 25 Kohlenstoffatomen, einen aromatischen Kohlenwasserstoff mit 6 bis 12 Kohlenstoffatomen, einen aliphatischen Alkohol mit 1 bis 12 Kohlenstoffatomen, ein Polyalkylenglykol mit 2 bis 6 Kohlenstoffatomen je Alkylen, einen Dialkylether mit 2 bis 20 Kohlenstoffatomen je Alkylrest, ein Polyalkylenglykoldialkylether mit 1 bis 6 Kohlenstoffatomen je Alkylen, ein Carbonsäuredialkylamid, Essigsäure(C₁-C₄)alkylester, ein Nitril, Dialkylsulfoxid, Dialkylsulfon, ein Imidazolidinon, ein Pyrollidon oder ein Gemisch derselben, einsetzen.

Mit gutem Erfolg kann man als Lösungsmittel Benzol, Toluol, ortho-Xylol, meta-Xylol, para-Xylol, ein technisches Gemisch isomerer Xylole, Ethylbenzol, Mesitylen, Methanol, Ethanol, n-Propanol, i-Propanol, n-Butanol, i-Butanol, Essigsäuremethylester, Essigsäureethylester, Essigsäurebutylester, Dimethylformamid, Diethylformamid, Dimethylacetamid, Diethylacetamid, Dimethylsulfoxid, Dimethylsulfon, Sulfolan, 1,3-Dimethylimidazolidin-2-on, N-Pyrrolidon oder ein Gemisch derselben, insbesondere Toluol, ortho-Xylol, meta-Xylol, para-Xylol, ein technisches Gemisch isomerer Xylole, Essigsäuremethylester, Essigsäureethylester, Essigsäurebutylester, Dimethylformamid, Dimethylacetamid, 1,3-Dimethylimidazolidin-2-on oder ein Gemisch derselben, bevorzugt Toluol, ortho-Xylol, meta-Xylol, para-Xylol, ein technisches Gemisch isomerer Xylole oder Essigsäurebutylester einsetzen.

In einer Reihe von Fällen hat es sich im ersten Schritt der Reaktionsfolge bewährt, die Umsetzung der Verbindung der Formel (1) mit dem sekundären Amin der Formel (2) bei 0 bis 100°C durchzuführen.

Der Vollständigkeit halber sei an dieser Stelle darauf aufmerksam gemacht, daß die in dem nachfolgenden zweiten Schritt durchzuführende Umsetzung in Gegenwart der gleichen Base und des gleichen Lösungsmittels wie im ersten Schritt erfolgen kann. Demzufolge ist es sinnvoll, die Umsetzungen im ersten und zweiten Schritt in Gegenwart ein und derselben Base und ein und desselben Lösungsmittels ablaufen zu lassen. Da die Base in dem ersten Schritt nicht verbraucht wird, sondern aus dem Hydrohalogenid freigesetzt und der in dem zweiten Schritt ablaufenden Umsetzung zugeführt werden kann, entfallen zusätzliche Arbeitsschritte zur Abtrennung respektive Aufarbeitung der Base und des Lösungsmittels.

In dem zweiten Schritt wird das aus dem ersten Schritt resultierende 2-Chlor-4-dialkylamino-5-fluornitrobenzol in Gegenwart einer Base und eines Edelmetallkatalysators hydriert. Hierbei wird die Nitrogruppe in eine NH₂-Gruppe umgewandelt und zugleich das in ortho-Stellung zur Nitrogruppe stehende Chlor, unter Bildung von Chlorwasserstoff abgespalten. Aufgabe der Base ist es, den freigesetzten Chlorwasserstoff zu binden.

Man setzt üblicherweise in den zweiten Schritt als Base diesselbe Base wie in ersten Schritt, nämlich das sekundäre Amin der Formel (2) oder ein tertiäres Amin, insbesondere das sekundäre Amin der Formel (2) oder ein Trialkylamin, dessen Reste gleich oder verschieden und 1 bis 25, insbesondere 6 bis 25, bevorzugt 8 bis 12 Kohlenstoffatome je Alkylrest aufweisen, bevorzugt Triisooctylamin, Trialkyl(C₈/C₁₀)amine, Tri-n-dodecylamin oder ein Gemisch derselben ein.

Besonders geeignet als Base für den zweiten Schritt der Reaktionsfolge sind Trialkylamine, die wasserunlösliche, in organischer Phase lösliche Hydrochloride bilden. Hierzu zählen die bereits im ersten Schritt der Reaktionsfolge genannten Trialkylamine mit 7 bis 12 Kohlenstoffatomen je Alkylrest, wobei die Alkylreste gleich oder verschieden sein können, insbesondere Triisooctylamin, Trialkyl(C₈/C₁₀)amine (ein Gemisch von Trialkylaminen mit 8 bis 10 Kohlenstoffatomen je Alkylrest, beispielsweise Hostarex A 327, ein Handelsprdukt der Hoechst AG), Tri-(Ndodecylamin).

Man setzt die Base in einer Menge von 50 bis 500, insbesondere 100 bis 250, bevorzugt 100 bis 130 Mol-%, bezogen auf Äquivalent abzuspaltendes Chlor ein.

Die Base hat die Aufgabe, den während der dechlorierenden Hydrierung abgespaltenen Chlorwasserstoff zu binden.

Man setzt als Edelmetallkatalysator einen Palladiumträgerkatalysator ein. Der Edelmetallkatalysator enthält 0,1 bis 25, insbesondere 0,5 bis 10, bevorzugt 1,0 bis 5,0 Gew.-% Palladium.

Der Edelmetallkatalysator enthält Aktivkohle, Calciumcarbonat, Bariumsulfat, Bimsstein, Tonerde, Kieselgur, Kieselgel, Aluminiumoxid oder ein Gemisch derselben, insbesondere Aktivkkohle, Kieselgur, Aluminiumoxid oder ein Gemisch derselben, bevorzugt Aktivkohle als Trägermaterial.

Man führt die Hydrierung in Gegenwart von Wasserstoff durch, wobei man die Umsetzung in einer Vielzahl von Fällen bei einem Druck von 2 bis 50 bar, insbesondere bei einem Druck von 5 bis 30 bar und bei einer Temperatur von 40 bis 140°C, insbesondere 60 bis 120°C durchführt.

Bei Durchführung des zweiten Schritts der Reaktionsfolge ist darauf zu achten, daß man ein Lösungsmittel verwendet, daß unter den Bedingungen der Hydrierung inert ist. Ungeeignet als Lösungsmittel sind chlorierte aliphatische oder aromatische Kohlenwasserstoffe, da diese unter den Reaktionsbedingungen mit Wasserstoff reagieren können. Diese Einschränkungen hinsichtlich des Lösungsmittels gilt lediglich für die Stufe der Hydrierung. Um einen Wechsel des Lösungsmittels zu vermeiden, ist es vorteilhaft, bereits in den ersten Schritt der Reaktionsfolge ein Lösungsmittel, das auch für den zweiten Schritt der Reaktionsfolge geeignet ist, einzusetzen.

Geeignete Lösungsmittel sind beispielsweise die bereits vorstehend genannten Solventien.

Verwendet man eine Base, die mit dem freigesetzten Chlorwasserstoff ein in der organischen Phase unlösliches Hydrochlorid bildet, so empfiehlt es sich, dieses Hydrochlorid, beispielsweise durch Filtration abzutrennen und die organische Phase anschließend, wie bereits erwähnt, mit einer wäßrigen Lösung einer Säure zu versetzen.

Aus dem abgetrennten Hydrochlorid kann in einem separaten Arbeitsschritt durch Zusatz einer wäßrigen Lauge die freie Base zurückgewonnen und in den Prozeß wiedereingesetzt werden.

Besonders günstig ist es, eine Base, die mit dem freigesetzten Chlorwasserstoff in Wasser unlösliche, jedoch in der organischen Phase lösliche Hydrochloride bildet, einzusetzen. Diese wasserunlöslichen Hydrochloride gelangen nämlich bei der nachfolgenden Behandlung der organischen, das Wertprodukt enthaltenden Phase mit einer wäßrigen Lösung einer Säure nicht in die wäßrige Phase, sondern verbleiben in der organischen Phase und werden mit dieser abgetrennt.

Die abgetrennte organische Phase, die neben dem Hydrochlorid der Base gegebenenfalls überschüssig eingesetzte Base und Lösungsmittel enthält, wird mit wäßriger Lauge behandelt, wodurch aus dem Hydrochlorid der Base die freie Base gebildet wird. Die freigesetzte Base verbleibt in der organischen Phase und kann, falls ein in Wasser nicht oder nur geringfügig lösliches Lösungsmittel in den ersten Schritt der Reaktionsfolge eingesetzt worden ist, zusammen mit dem Lösungsmittel abgetrennt und, gegebenenfalls nach einer Zwischenreinigung, wieder in das erfindungsgemäße Verfahren eingesetzt werden. Dadurch kann das als Base verwendete tertiäre Amin ohne nennenswerte Verluste im Kreis geführt werden. Ein Verbrauch an Hilfsstoffen findet lediglich bei der Freisetzung der Base aus ihren Salzen statt, wobei eine entsprechende Menge Alkali in Form einer wäßrigen NaOH oder KOH Lösung verbraucht wird.

Das nach erfolgter Hydrierung anfallende Reaktionsgemisch wird gegebenenfalls nach Abtrennung des Edelmetallkatalysators in einem dritten Schritt mit einer wäßrigen Lösung einer Säure versetzt, wobei aus dem Reaktionsgemisch ein wasserlösliches Salz des entsprechenden 3-Fluor-4-dialkylaminoanilins gebildet und in die wäßrige Phase überführt wird.

Als Säure setzt man eine Mineralsäure, beispielsweise Salzsäure, Schwefelsäure, insbesondere Salzsäure ein. Die wäßrige Lösung der Säure enthält üblicherweise 1 bis 30, insbesondere 5 bis 15 Gew.-% Säure.

Man trennt anschließend die wäßrige Phase von der organischen Phase und gegebenenfalls vom Edelmetallkatalysator ab, der - falls auf eine vorangehende Abtrennung verzichtet wird - nunmehr zu entfemen ist.

Es ist nicht erforderlich, an dieser Stelle des Verfahrens das Wertprodukt (3-Fluor-4-dialkylaminoanilin) zu isolieren, um es in isolierter Form weiterzuverarbeiten. Es stellt im Gegenteil einen Vorteil des erfindungsgemäßen Verfahrens dar, an dieser Stelle auf eine Isolierung des Wertproduktes, beispielsweise durch Kristallisation und/oder Destillation zu verzichten und statt dessen lediglich eine Phasentrennung durchzuführen.

Anschließend setzt man das in Wasser gelöste Salz des 3-Fluor-4-dialkylaminoanilins in Gegenwart der basischen Verbindung mit dem Chlorameisensäureester um.

Als basische Verbindung kann man ein Oxid, Carbonat oder Hydrogencarbonat eines Alkalimetalls oder Erdalkalimetalls oder ein Amin oder ein Gemisch derselben, insbesondere ein Alkalimetallhydrogencarbonat, ein Alkalimetallcarbonat oder ein Gemisch derselben, bevorzugt ein Alkalimetallhydrogencarbonat oder ein Gemisch derselben einsetzen. Es hat sich besonders bewährt, Natrium- und/oder Kaliumhydrogencarbonat zu verwenden.

Die basische Verbindung kann in Substanz oder in Form einer Lösung, insbesondere in Form einer wäßrigen Lösung, verwendet werden.

Üblicherweise setzt man je Mol Ameisensäureester der Formel (3): 0,5 bis 3, insbesondere 1,5 bis 2,5, bevorzugt 2 bis 2,2 Äquivalente basischer Verbindung ein. Es hat sich häufig als günstig erwiesen, je Mol Ameisensäureester der Formel (3) 2 bis 3 Äquivalente basischer Verbindung einzusetzen.

Geeignete Chlorameisensäureester sind beispielsweise Chlorameisensäurealkylester mit 1 bis 4 Kohlenstoffatomen im Alkylrest, Chlorameisensäurebenzylester, insbesondere Chlorameisensäureemethylester, Chlorameisensäureethylester, Chlorameisensäure-n-propylester, Chlorameisensäure-i-propyl-ester, Chlorameisensäure-n-butylester, Chlorameisen-ibutylester, Chlorameisensäurebenzylester, bevorzugt Chlorameisensäurebenzylester.

Die basische Verbindung dient dazu, aus dem wasserlöslichen Salz das entsprechende 3-Fluor-4-dialkylaminoanilin freizusetzen, den bei der Umsetzung entstehenden Chlorwasserstoff zu binden und gegebenenfalls noch vorhandene überschüssige Säure zu neutralisieren. Auf diese Weise wird zudem sichergestellt, daß nicht Salze des N-Carboxyalkyl-3-fluor-4-dialkylaminoanilins, die möglicherweise wasserlöslich sind und in der wäßrigen Lösung verbleiben würden, gebildet werden. Dies erleichtert die Abtrennung des gewünschten N-Carboxyalkyl-3-fluor-4-dialkylaminoanilins aus der wäßrigen Phase, beispielsweise durch Filtration.

Nach einer besonderen Verfahrensvariante legt man die wäßrige Lösung des wasserlöslichen Salzes des 3-Fluor-4-dialkylaminoanilins vor und führt den Chlorameisensäureester und die basische Verbindung gleichzeitig, aber voneinander getrennt unter guter Vermischung zu. Diese Variante besitzt den Vorzug, sich besonders einfach durchführen zu lassen.

Da das anfallende Aminhydrochlorid üblicherweise wasserlöslich ist, kann bei dieser Variante in besonders vorteilhafter Weise auf die Gegenwart eines organischen Lösungsmittels verzichtet werden.

In den meisten Fällen erweist es sich als ausreichend, die Umsetzung mit dem Chlorameisensäureester bei 10 bis 80°C durchzuführen.

Nach erfolgter Umsetzung erhält man das N-Carboxyalkyl-3-fluor-4-dialkylaminoanilin üblicherweise als Feststoff, der sich aus der wäßrigen Phase abscheidet und durch Filtration und/oder Extraktion mittels eines organischen, in Wasser unlöslichen Solvens abgetrennt werden kann. Falls gewünscht, kann das Wertprodukt noch einer weiteren Reinigung zugeführt werden. In der Regel fällt das Wertprodukt jedoch in ausreichend hoher Reinheit an.

Das Verfahren läßt sich unter reduziertem Druck, Atmosphärendruck oder erhöhtem Druck durchführen.

Es eignet sich sowohl für eine kontinuierliche als auch diskontinuierliche Arbeitsweise.

Die vorliegende Erfindung betrifft femer die Verbindungen 2-Chlor-4-diethylamino-5-fluornitrobenzol, 2-Chlor-5-fluor-4-morpholinonitrobenzol, 2-Chlor-5-fluor-4-piperidino-nitrobenzol und 2-Chlor-5-fluor-4-piperazinonitrobenzol, insbesondere 2-Chlor-5-fluor-4-morpholinonitrobenzol und 2-Chlor-5-fluor-4-piperazinonitrobenzol.

Die vorliegende Erfindung betrifft darüberhinaus die Verbindungen 2-Chlor-5-fluor-4-morpholinoanilin und 2-Chlor-5-fluor-4-piperazinoanilin.
Von besonderem Interesse sind die Verbindungen 2-Chlor-5-fluor-4-morpholinonitrobenzol und 2-Chlor-5-fluor-4-morpholinoanilin.

Sämtliche der vorstehend genannten Verbindungen stellen wertvolle Zwischenprodukte zur Herstellung von N-Carboxyalkyl-3-fluor-4-dialkylaminoanilinen dar.

Die nachfolgenden Beispiele beschreiben die Erfindung näher, ohne sie zu beschränken.

### Experimenteller Teil

### Beispiel 1

### Herstellung von N-Carboxybenzyl-3-fluor-4-morpholinoanilin

Man legt in einem 1000 ml Dreihalskolben, der mit Rührer, Thermometer und Rückflußkühler bestückt ist, 58,1 g (0,3 mol) 2-Chlor-4,5-difluornitrobenzol, 120 ml Toluol und 180 g (etwa 0,45 mol) Tri-N(octyl/decyl)amin (ein Gemisch von Trialkyl(C₈/C₁₀)-aminen) unter Rühren vor, erwärmt auf 50°C und setzt innerhalb von einer Stunde 31,3 g (0,36 mol) Morpholin zu.

Anschließend rührt man bei 50°C noch 5 Stunden nach, setzt 666 g Toluol zu und erhitzt auf 80°C. Dann setzt man unter guter Vermischung 66 g einer 20 Gew.-%igen wäßrigen Natronlauge zu und trennt anschließend die wäßrige Phase ab.

Die nach Abtrennung der wäßrigen Phase erhaltene organische Phase wird zusammen mit 3,9 g eines Palladiumkatalysators (5 Gew.-% Palladium auf Aktivkohle, 50 Gew.-% wasserfeucht) unter Stickstoffschutz in einen 2 I Rührautoklaven überführt. Der Autoklav wird unter Stickstoffatmosphäre gehalten und nach Befüllung geschlossen.

Man erhitzt unter Rühren auf 95°C und setzt bei dieser Temperatur Wasserstoff mit einem Druck von 30 bar solange zu, bis kein Wasserstoff mehr aufgenommen wird. Der Autoklav wird entspannt und der Katalysator wird durch Absaugen mittels einer Nutsche abgetrennt.

Die nach Abtrennung des Katalysators anfallende Reaktionslösung wird mit einer aus 65 g einer 37 Gew.-%igen Salzsäure und 420 g Wasser hergestellten Lösung unter Mischen versetzt, wobei das gewünschte Produkt in die wäßrige Phase überführt wird. Die wäßrige Phase wird anschließend bei Raumtemperatur abgetrennt und innerhalb einer Stunde getrennt, aber gleichzeitig mit 693 g (0,66 mol) NaHCO₃ (wäßrige Lösung mit 8 Gew.-% NaHCO₃) und 56,0 g (0,33 mol) Chlorameisensäurebenzylester versetzt.

Nach Ende der Zugabe wird noch eine Stunde nachgerührt. Dann wird der gebildete Feststoff abgesaugt, mit Wasser gewaschen und anschließend getrocknet.

Alle Reaktionsschritte werden unter Inertgas-Schutz (Stickstoff) durchführt.

Man erhält 82,4 g (0,25 mol) N-Carboxybenzyl-3-fluor-4-morpholinoanilin. Das entspricht einer Ausbeute von 83,3 %, bezogen auf eingesetztes 2-Chlor-4,5-difluornitrobenzol.

### Beispiel 1a

### Herstellung von 2-Chlor-5-fluor-4-morpholinonitrobenzol

Man legt in einem 100 ml Dreihalskolben, der mit Tropftrichter, KPG-Rührer und Rückflußkühler bestückt ist, eine Lösung von 4,8 g (25 mmol) 2-Chlor-4,5-difluornitrobenzol in 10,6 g Toluol vor.
Zu dieser Lösung tropft man bei Raumtemperatur und unter Rühren innerhalb 1 Stunde 4,4 g (50 mmol) Morpholin. Anschließend rührt man 2 Stunden bei Raumtemperatur nach und saugt die bei der Umsetzung gebildeten Reaktionsprodukte ab.
Der Filterkuchen wird dreimal mit je 10 ml Wasser gewaschen und anschließend getrocknet.
Man erhält 5,7 g (22,1 mmol) 2-Chlor-5-fluor-4-morpholinonitrobenzol. Dies entspricht einer Ausbeute von 89,1 %, bezogen auf eingesetztes 2-Chlor-5-fluor-4-morpholinonitrobenzol.
2-Chlor-5-fluor-4-morpholinonitrobenzol der nachstehenden Formel 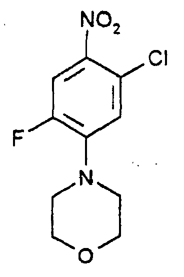 besitzt einen Schmelzpunkt von 164,9°C.
¹H-NMR: δ (TMS) = 0, (CDCl₃) : δ = 3,28 (br, t); 3,87 (br, t, 4,7 Hz); 6,93 (d, J_{F,C} = 7,8 Hz); 7,81 (d, J_{F,C} = 12,8 Hz)
¹³C-NMR: δ (CDCl₃) = 77, (CDCl₃) : δ = 49,63 (t, J_{F,C} = 5,14Hz); 66,38 (t, J_{F,C} < 0,7 Hz); 114,97 (d, J_{F,C} = 27,76 Hz); 119,76 (d, J_{F,C} = 3,90 Hz); 124,92 (s, J_{F,C} = 3,17Hz); 138,51 (br, s, J_{F,C} = 8,21Hz); 144,27 (s, J_{F,C} = 8,45Hz); 151,27 (s, J_{F,C} = 250,05 Hz)
¹⁹F-NMR: δ [CFCl₃ (virt. int.)] = 0, (CDCl₃) : δ = -121,14 (s)

### Beispiel 1b

### Herstellung von 2-Chlor-5-fluor-4-morpholinoanilin

In einen 2 I Rührautoklaven werden 78,2 g (0,3 mol) 2-Chlor-5-fluor-4-morpholinonitrobenzol zusammen mit 3,9 g eines Platin-Aktivkohle-Katalysators (5 Gew.-% Pt, 50 Gew.-% Wassergehalt, sulfitiert) in 782 g (10,2 mol) Toluol bei einer Temperatur von 70°C vorgelegt. Der Autoklav wird geschlossen und mit Stickstoff inertisiert. Die Reaktionstemperatur wird auf 80°C gesteigert und bei dieser Temperatur wird ein Wasserstoffdruck von 5 bar solange gehalten, bis die Wasserstoffaufnahme stark abfällt.

Nach dem Absaugen des Katalysators, wird das Reaktionsprodukt 2-Chlor-5-fluor-4-morpholinoanilin ohne weitere Reinigung für die Folgestufe weiterverwendet.

Alternativ wird das Lösungsmittel eingeengt, 2-Chlor-5-fluor-4-morpholinoanilin ausgefällt und gereinigt. Man erhält auf diese Weise 57,4 g (0,25 mol) 2-Chlor-5-fluor-4-morpholinoanilin, entsprechend 83,3 % der theoretischen Ausbeute, bezogen auf eingesetztes 2-Chlor-5-fluor-4-morpholinonitrobenzol.
Schmelzpunkt: 89,5°C
¹H-NMR: δ (TMS) = 0, (CDCl₃): δ = 2,95 (m_{c}); 3,84 (m_{c}); 3,90 (br, s); 6,51 (d, J_{F,H} = 13,0 Hz), 6,87 (d, J_{F,H} = 8,4 Hz).
¹³C-NMR: δ (CDCl₃) = 77, (CDCl₃): δ = 51,59 (t, J_{F,C} = 2,6 Hz); 67,00 (t); 104,15 (d, J_{F,C} = 25,5 Hz); 113,90 (s, J_{F,C} = 3,0 Hz); 120,29 (d, J_{F,C} = 4,4 Hz); 132,20 (s, J_{F,C} = 11,1 Hz); 138,78 (s, J_{F,C} = 10,8 Hz); 155,39 (s, J_{F,C} = 245,4 Hz).

### Beispiel 2

### Herstellung von N-Carboxybenzyl-3-fluor-4-morpholinoanilin ausgehend von 2,4-Dichlor-5-fluornitrobenzol

Man legt in einem 1000 ml Dreihalskolben der mit Rührer, Thermometer und Rückflußkühler bestückt ist, 130,0 g (0,62 mol) 2,4-Dichlor-5-fluornitrobenzol und 260 ml N,N-Dimethylacetamid unter Rühren vor und setzt bei 25°C innerhalb von 2 Stunden 113,3 g (1,3 mol) Morpholin zu.

Anschließend rührt man bei 25°C noch 16 Stunden nach. Danach destilliert man bei 25 mbar insgesamt 235 ml Lösungsmittel (N,N-Dimethylacetamid) ab. Der verbleibende 2-Chlor-5-fluor-4-morpholinonitrobenzol enthaltende Rückstand wird mittels einer Filternutsche scharf abgesaugt und mit Toluol und Wasser gewaschen und getrocknet.

Der getrocknete, 2-Chlor-5-fluor-4-morpholinonitrobenzol bereits in einer Reinheit ≥ 95 % enthaltende Rückstand wird in 666 g Toluol aufgenommen und nach Zugabe von 3,9 g eines Palladiumkatalysators (5 Gew.-% Palladium auf Aktivkohle, 50 Gew.-% wasserfeucht), wie in Beispiel 1 beschrieben, weiterverarbeitet.

Man erhält 79,6 g (0,24 mol) N-Carboxybenzyl-3-fluor-4-morpholinoanilin. Dies entspricht einer Ausbeute von 48,2 %, bezogen auf eingesetztes 2,4-Dichlor-5-nitrobenzol.

### Beispiel 3

### Herstellung von N-Carboxybenzyl-3-fluor-4-piperazinoanilin

Man legt in einem 1000 ml Dreihalskolben, der mit Rührer, Thermometer und Rückflußkühler bestückt ist, 97,0 g (0,5 mol) 2-Chlor-4,5-difluornitrobenzol und 284 g Toluol unter Rühren vor, erwärmt auf 50°C und setzt innerhalb von einer Stunde 94,8 g (1,1 mol) Piperazin zu.

Anschließend rührt man bei 50°C noch 2 Stunden nach und erhitzt auf 80°C. Dann setzt man unter guter Durchmischung 200 g einer 10 Gew.%-igen wäßrigen Natronlauge zu und trennt anschließend die wäßrige Phase ab.

Die nach der Abtrennung der wäßrigen Phase erhaltene organische Phase wird zusammen mit 3,9 g eines Palladiumkatalysators (5 Gew.-% Palladium auf Aktivkohle, 50 Gew.-% wasserfeucht) und 300 g Tri-N-(octyl-/decyl)-amin (ein Gemisch aus Trialkyl(C₈/C₁₀)-aminen, unter Stickstoff in einen 21 Autoklaven überführt. Der Autoklav wird unter Stickstoff gehalten und nach Befüllung geschlossen.

Man erhitzt unter Rühren auf 95°C und setzt bei dieser Temperatur Wasserstoff mit einem Druck von 30 bar solange zu, bis kein Wasserstoff mehr aufgenommen wird. Der Autoklav wird entspannt und der Katalysator mittels einer Nutsche abgetrennt.

Die nach Abtrennung des Katalysators, anfallende Reaktionslösung wird mit insgesamt 547 g 10%-iger wäßriger Salzsäure extrahiert und die wäßrige Phase abgetrennt.

Die wäßrige Phase wird bei Raumtemperatur innerhalb einer Stunde gleichzeitig, aber getrennt mit 1940 g (0,44 mol) NaHCO₃ (10%-ige wäßrige Lösung) und 187,6 g (1,1 mol) Chlorameisensäurebenzylester versetzt.

Nach beendeter Zugabe wird noch eine Stunde nachgerührt. Dann wird der gebildete Feststoff abgesaugt, mit Wasser gewaschen und anschließend getrocknet.

Alle Reaktionsschritte werden unter Inertgas-Schutz (Stickstoff) durchgeführt.

Man erhält 187,5 g (0,4 mol) N-Carboxybenzyl-3-fluor-4-piperazinoanilin. Das entspricht einer theoretischen Ausbeute von 81,0 %, bezogen auf eingesetztes 2-Chlor-4, 5-difluornitrobenzol.

### Beispiel 3a

### Herstellung von 2-Chlor-5-fluor-4-piperazinonitrobenzol

Man legt in einem 200 ml Dreihalskolben, der mit Tropftrichter, KPG-Rührer und
Rückflußkühler bestückt ist, eine Lösung von 19,3 g (0,08 mol) 2-Chlor-4,5-difluornitrobenzol in 100 g Toluol vor.
Zu dieser Lösung tropft man bei Raumtemperatur und unter Rühren innerhalb 1 Stunde 18,1 g (0,21 mol) Piperazin. Anschließend rührt man 2 Stunden bei Raumtemperatur nach. Die bei der Umsetzung gebildete Reaktionslösung wird dreimal mit je 20 ml Wasser gewaschen und getrocknet.
Nach Entfernen des Lösungsmittels erhält man 18,5 g (0,071 mmol) 2-Chlor-5-fluor-4-piperazinonitrobenzol. Dies entspricht einer Ausbeute von 95 %, bezogen auf eingesetztes 2-Chlor-4,5-difluomitrobenzol.
2-Chlor-5-fluor-4-piperazinonitrobenzol der nachstehenden Formel 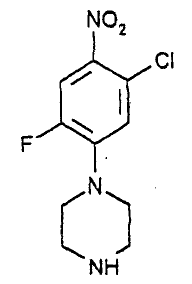 besitzt einen Schmelzpunkt von 91,5°C (Reinheit: 92,9%, ermittelt durch HPLC).
¹H-NMR: δ (TMS) = 0, (CDCl₃) : δ = 1,81 (d); 3,04 (m_{c}); 3,26 (m_{c}); 6,91 (d, J_{F,C} = 7,9 Hz); 7,80 (d, J_{F,C} = 12,9 Hz).
¹³C-NMR: δ (CDCl₃) = 77, (CDCl₃) : δ = 45,81 (t); 50,71 (t, J_{F,C} = 5,2Hz); 114,97 (d, J_{F,C} = 27,9 Hz); 119,91 (d, J_{F,C} = 4,1 Hz); 124,95 (s, J_{F,C} = 3,1 Hz); 138,51 (s, J_{F,C} = 8,5Hz); 144,80 (s, J_{F,C} = 8,4Hz); 151,24 (s, J_{F,C} = 249,9 Hz).

### Beispiel 3b

### Herstellung von 2-Chlor-5-fluor-4-piperazinoanilin

Man setzt 77,8 g (0,3 mol) 2-Chlor-5-fluor-4-piperazinonitrobenzol zusammen mit 3,9 g eines Platin-Aktivkohle-Katalysators (5 Gew.-% Pt; 50 Gew.-% Wassergehalt, sulfitiert) in 782 g (10,2 mol) Toluol ein und arbeitet wie in Beispiel 1b beschrieben.

Man erhält 54,1 g (0,24 mol) 2-Chlor-5-fluor-4-piperazinoanilin entsprechend 78,2 % der theoretischen Ausbeute, bezogen auf eingesetztes 2-Chlor-5-fluor-4-piperazinonitrobenzol.
Schmelzpunkt: 110°C (95 %-ig)
¹H-NMR: δ (TMS) = 0, (CDCl₃): δ = 1,67 (m_{c}); 3,88 (m_{c}), 3,90 (br, s); 6,50 (d, J_{F,H} = 13,0 Hz), 6,87 (d, J_{F,H} = 8,4 Hz).
¹³C-NMR: δ (CDCl₃) = 77, (CDCl₃): δ = 46,24 (t, J_{F,C} = 0 Hz); 52,62 (t, J_{F,C} = 2,3 Hz); 104,10 (d, J_{F,C} = 25,6 Hz); 113,85 (s, J_{F,C} = 2,9 Hz); 120,47 (d, J_{F,C} = 4,4 Hz); 132,94 (s, J_{F,C} = 10,8 Hz); 138,52 (s, J_{F,C} = 10,7 Hz); 155,40 (s, J_{F,C} = 245,4 Hz).

### Beispiel 4

### Herstellung von 2-Chlor-4-diethylamino-5-fluornitrobenzol

Man legt in einem 100 ml Dreihalskolben, der mit Tropftrichter, KPG-Rührer und
Rückflußkühler bestückt ist, eine Lösung von 4,8 g (25 mmol) 2-Chlor-4,5-difluornitrobenzol in 14,4 g Toluol vor.
Zu dieser Lösung tropft man bei Raumtemperatur und unter Rühren innerhalb 1 Stunde 5,7 g (78 mmol) Diethylamin. Anschließend rührt man 2 Stunden bei Raumtemperatur nach. Die bei der Umsetzung gebildete Reaktionslösung wird dreimal mit je 35 ml Wasser gewaschen und getrocknet.
Nach Entfernen des Lösungsmittels erhält man 5,4 g (22 mmol) 2-Chlor-4-diethylamino-5-fluoronitrobenzol. Dies entspricht einer Ausbeute von 88 %, bezogen auf eingesetztes 2-Chlor-4,5-difluornitrobenzol.
Schmelzpunkt: 68,9°C
¹H-NMR: δ (TMS) = 0, (CDCl₃) : δ = 1,24 (td, J_{F,H} = 0,5 Hz); 3,44 (qd, J_{F,H} = 1,5 Hz); 6,72 (d, J_{F,H} = 8,3 Hz); 7,83 (d, J_{F,H} = 14,6Hz).
¹³C-NMR: δ (CDCl₃) = 77, (CDCl₃) : δ = 13,04 (q, J_{F,C} = 1,9 Hz); 46,42 (t, J_{F,C} = 6,2 Hz); 115,70 (d, J_{F,C} = 29,2 Hz); 117,06 (d, J_{F,C} = 5,3 Hz); 125,67 (s, J_{F,C} = 2,3 Hz); 134,37 (s, J_{F,C} = 8,0 Hz); 142,25 (s, J_{F,C} = 8,3Hz); 148,48 (s, J_{F,C} = 245,9Hz).

### Beispiel 5

### Herstellung von 2-Chlor-5-fluor-4-piperidinonitrobenzol

Man legt in einem 100 ml Dreihalskolben, der mit Tropftrichter, KPG-Rührer und Rückflußkühler bestückt ist, eine Lösung von 4,8 g (25 mmol) 2-Chlor-4,5-difluomitrobenzol in 14,4 g Toluol vor.
Zu dieser Lösung tropft man bei Raumtemperatur und unter Rühren innerhalb 1 Stunde 4,3 g (50 mmol) Piperidin. Anschließend rührt man 2 Stunden bei Raumtemperatur nach. Die bei der Umsetzung gebildete Reaktionslösung wird dreimal mit je 35 ml Wasser gewaschen und getrocknet.
Nach Entfernen des Lösungsmittels erhält man 5,3 g (21 mmol) 2-Chlor-5-fluor-4-piperidinonitrobenzol. Dies entspricht einer Ausbeute von 82,0 %, bezogen auf eingesetztes 2-Chlor-4,5-difluornitrobenzol.

2-Chlor-5-fluor-4-piperidinonitrobenzol der nachstehenden Formel 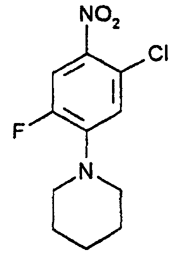 besitzt einen Schmelzpunkt von 70°C (Reinheit: 99,5%, ermittelt durch HPLC).
¹H-NMR: δ (TMS) = 0, (CDCl₃): δ = 1,66 (m_{c}); 1,72 (m_{c}); 3,27 (t, 5,4 Hz); 6,90 (d, J_{F,H} = 8,0 Hz); 7,79 (d, J_{F,H} = 13,1 Hz).
¹³C-NMR: δ (CDCl₃) = 77, (CDCl₃) : δ = 23,96 (t, J_{F,C} = 0,8 Hz); 25,66 (t); 114,97 (d, J_{F,C} = 28,1 Hz); 119,91 (d, J_{F,C} = 4,2 Hz); 125,01 (s, J_{F,C} = 2,9 Hz); 137,39 (s, J_{F,C} = 8,5Hz); 145,11 (s, J_{F,C} = 8,4 Hz); 151,04 (s, J_{F,C} = 249,4 Hz).

## Patentansprüche

1. Verfahren zur Herstellung von N-Carboxyalkyl-3-fluor-4-dialkylaminoanilinen, **dadurch gekennzeichnet, daß** man in einem ersten Schritt ein ortho-Nitrochlorbenzol der Formel (1) 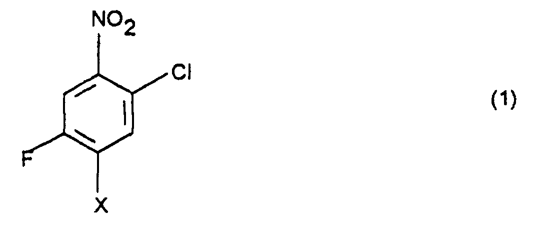 worin X für Cl oder F steht, mit einem sekundären Amin der Formel (2) HNR¹R², worin R¹ und R² unabhängig voneinander gleich oder verschieden sind und für einen Alkylrest mit 1 bis 10 Kohlenstoffatomen stehen oder zusammen mit dem N-Atom, an dem sie stehen, einen Ring mit 3 bis 7 Gliedern bilden, in Gegenwart einer Base in Anwesenheit oder Abwesenheit eines Lösungsmittels bei -10 bis 120°C umsetzt, in einem zweiten Schritt das 2-Chlor-4-dialkylamino-5-fluornitrobenzol in Gegenwart einer Base und eines Edelmetallkatalysators mit Wasserstoff bei 30 bis 150°C und 1 bis 100 bar umsetzt, in einem dritten Schritt aus dem Reaktionsgemisch das 3-Fluor-4-dialkylaminoanilin mit einer wäßrigen Lösung einer Säure als in Wasser gelöstes Salz extrahiert, die wäßrige Phase abtrennt und das in Wasser gelöste Salz des 3-Fluor-4-dialkylaminoanilins in Gegenwart einer basischen Verbindung mit einem Chlorameisensäureester der Formel (3) CICO₂R³, worin R³ für einen Alkylrest mit 1 bis 10 Kohlenstoffatomen oder Aralkylrest mit 7 bis 20 Kohlenstoffatomen steht, bei 0 bis 100°C umsetzt.

2. Verfahren nach Anspruch 1, **dadurch gekennzeichnet, daß** man als sekundäres Amin der Formel (2) Dimethylamin, Diethylamin, Di-n-propylamin, Di-i-propylamin, Di-n-butylamin, Di-i-butylamin, Piperidin, Morpholin oder Piperazin einsetzt.

3. Verfahren nach Anspruch 1 oder 2, **dadurch gekennzeichnet, daß** man in den ersten Schritt als Base das sekundäre Amin der Formel (2) oder ein tertiäres Amin einsetzt.

4. Verfahren nach einem oder mehreren der Ansprüche 1 bis 3, **dadurch gekennzeichnet, daß** man in den ersten Schritt als Base ein Trialkylamin, dessen Reste gleich oder verschieden sind und 1 bis 25 Kohlenstoffatome je Alkylrest aufweisen, einsetzt.

5. Verfahren nach einem oder mehreren der Ansprüche 1 bis 4, **dadurch gekennzeichnet, daß** man in den ersten Schritt die Base in einer Menge von 50 bis 500 Mol-%, bezogen auf Äquivalent abzuspaltendes Chlor oder Fluor, einsetzt.

6. Verfahren nach einem oder mehreren der Ansprüche 1 bis 5, **dadurch gekennzeichnet, daß** man als Lösungsmittel einen aliphatischen Kohlenwasserstoff mit 5 bis 25 Kohlenstoffatomen, einen aromatischen Kohlenwasserstoff mit 6 bis 12 Kohlenstoffatomen, einen aliphatischen Alkohol mit 1 bis 12 Kohlenstoffatomen, ein Polyalkylenglykol mit 2 bis 6 Kohlenstoffatomen je Alkylen, einen Dialkylether mit 2 bis 20 Kohlenstoffatomen je Alkylrest, ein Polyalkylenglykoldialkylether mit 1 bis 6 Kohlenstoffatomen je Alkylen, ein Carbonsäuredialkylamid, einen Essigsäure(C₁-C₄)alkylester, ein Nitril, ein Dialkylsulfoxid, ein Dialkylsulfon, ein Imidazolinon, ein Pyrrolidon oder ein Gemisch derselben einsetzt.

7. Verfahren nach einem oder mehreren der Ansprüche 1 bis 6, **dadurch gekennzeichnet, daß** man in den zweiten Schritt als Base das sekundäre Amin der Formel (2) oder ein tertiäres Amin einsetzt.

8. Verfahren nach einem oder mehreren der Ansprüche 1 bis 7, **dadurch gekennzeichnet, daß** man in den zweiten Schritt als Base ein Trialkylamin, dessen Reste gleich oder verschieden sind und 1 bis 25 Kohlenstoffatome je Alkylrest aufweisen, einsetzt.

9. Verfahren nach einem oder mehreren der Ansprüche 1 bis 8, **dadurch gekennzeichnet, daß** man in den zweiten Schritt die Base in einer Menge von 50 bis 500 Mol%, bezogen auf Äquivalent abzuspaltendes Chlor, einsetzt.

10. Verfahren nach einem oder mehreren der Ansprüche 1 bis 9, **dadurch gekennzeichnet, daß** man als Edelmetallkatalysator einen Palladium-Träger-Katalysator einsetzt.

11. Verfahren nach einem oder mehreren der Ansprüche 1 bis 10, **dadurch gekennzeichnet, daß** als Säure eine Mineralsäure eingesetzt wird.

12. Verfahren nach einem oder mehreren der Ansprüche 1 bis 11, **dadurch gekennzeichnet, daß** man als basische Verbindung ein Oxid, Carbonat oder Hydrogencarbonat eines Alkalimetalls oder Erdalkalimetalls oder ein Amin oder ein Gemisch derselben einsetzt.

13. Verfahren nach einem oder mehreren der Ansprüche 1 bis 12, **dadurch gekennzeichnet, daß** man je Mol Chlorameisensäureester der Formel (3): 0,5 bis 3 Äquivalente basischer Verbindung einsetzt.

14. Die Verbindungen 2-Chlor-4-diethylamino-5-fluomitrobenzol, 2-Chfor-5-fluor-4-morpholinonitrobenzol, 2-Chlor-5-fluor-4-piperidinonitrobenzol und 2-Chlor-5-fluor-4-piperazinonitrobenzol.

15. Die Verbindungen 2-Chlor-5-fluor-4-morpholinoanilin und 2-Chlor-5-fluor-4-piperazinoanilin.

## Claims

1. A process for the preparation of N-carboxyalkyl-3-fluoro-4-dialkylaminoanilines, which comprises reacting, in a first step, an ortho-nitrochlorobenzene of the formula (1) 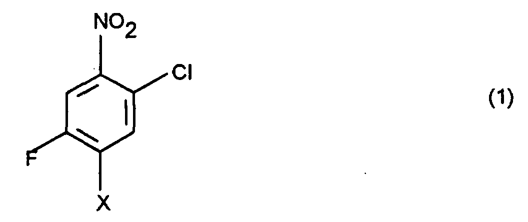 in which X is Cl or F, with a secondary amine of the formula (2) HNR¹R², in which R¹ and R², independently of one another, are identical or different and are an alkyl radical having from 1 to 10 carbon atoms or, together with the N atom to which they are bonded, form a ring having from 3 to 7 members, in the presence of a base in the presence or absence of a solvent at from -10 to 120°C, reacting, in a second step, the 2-chloro-4-dialkylamino-5-fluoronitrobenzene with hydrogen at from 30 to 150°C and from 1 to 100 bar in the presence of a base and a noble-metal catalyst and, in a. third step, extracting the 3-fluoro-4-dialkylaminoaniline from the reaction mixture using an aqueous solution of an acid as a salt dissolved in water, removing the aqueous phase and reacting the 3-fluoro-4-dialkylaminoaniline salt, dissolved in water, with a chloroformate of the formula (3) ClCO₂R³, in which R³ is an alkyl radical having from 1 to 10 carbon atoms or an aralkyl radical having from 7 to 20 carbon atoms, at from 0 to 100°C in the presence of a basic compound.

2. The process as claimed in claim 1, wherein the secondary amine of the formula (2) is dimethylamine, diethylamine, di-n-propylamine, di-i-propylamine, di-n-butylamine, di-i-butylamine, piperidine, morpholine or piperazine.

3. The process as claimed in claim 1 or 2, wherein the base used in the first step is the secondary amine of the formula (2) or a tertiary amine.

4. The process as claimed in one or more of claims 1 to 3, wherein the base used in the first step is a trialkylamine whose radicals are identical or different and contain from 1 to 25 carbon atoms per alkyl radical.

5. The process as claimed in one or more of claims 1 to 4, wherein the base is used in the first step in an amount of from 50 to 500 mol-%, based on the number of equivalents of chlorine or fluorine to be eliminated.

6. The process as claimed in one or more of claims 1 to 5, wherein the solvent used is an aliphatic hydrocarbon having from 5 to 25 carbon atoms, an aromatic hydrocarbon having from 6 to 12 carbon atoms, an aliphatic alcohol having from 1 to 12 carbon atoms, a polyalkylene glycol having from 2 to 6 carbon atoms per alkylene, a dialkyl ether having from 2 to 20 carbon atoms per alkyl radical, a polyalkylene glycol dialkyl ether having from 1 to 6 carbon atoms per alkylene, a dialkylcarboxamide, a (C₁-C₄)alkyl acetate, a nitrile, a dialkyl sulfoxide, a dialkyl sulfone, an imidazolinone, a pyrrolidone or a mixture thereof.

7. The process as claimed in one or more of claims 1 to 6, wherein the base used in the second step is the secondary amine of the formula (2) or a tertiary amine.

8. The process as claimed in one or more of claims 1 to 7, wherein the base used in the second step is a trialkylamine whose radicals are identical or different and contain from 1 to 25 carbon atoms per alkyl radical.

9. The process as claimed in one or more of claims 1 to 8, wherein the base is used in the second step in an amount of from 50 to 500 mol-%, based on the number of equivalents of chlorine to be eliminated.

10. The process as claimed in one or more of claims 1 to 9, wherein the noble-metal catalyst used is a supported palladium catalyst.

11. The process as claimed in one or more of claims 1 to 10, wherein the acid used is a mineral acid.

12. The process as claimed in one or more of claims 1 to 11, wherein the basic compound used is an oxide, carbonate or hydrogencarbonate of an alkali metal or alkaline earth metal or an amine or a mixture thereof.

13. The process as claimed in one or more of claims 1 to 12, wherein from 0.5 to 3 equivalents of basic compound are used per mole of chloroformate of the formula (3).

14. The compounds 2-chloro-4-diethylamino-5-fluoronitrobenzene, 2-chloro-5-fluoro-4-morpholinonitrobenzene, 2-chloro-5-fluoro-4-piperidinonitrobenzene and 2-chloro-5-fluoro-4-piperazinonitrobenzene.

15. The compounds 2-chloro-5-fluoro-4-morpholinoaniline and 2-chloro-5-fluoro-4-piperazinoaniline.

## Revendications

1. Procédé pour la préparation de N-carboxyalkyl-3-fluoro-4-dialkylaminoanilines, **caractérisé en ce qu'**on met à réagir dans une première étape un orthonitrochlorobenzène de formule (1) 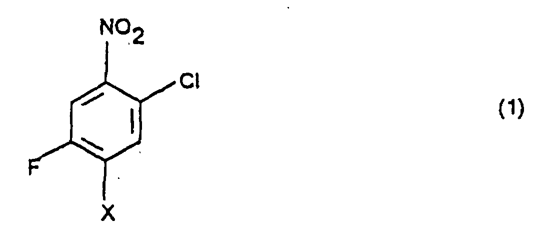 dans laquelle X représente Cl ou F, avec une amine secondaire de formule (2) HNR¹R², dans laquelle R¹ et R² sont, indépendamment l'un de l'autre, identiques ou différents et représentent un groupe alkyle ayant de 1 à 10 atomes de carbone ou forment ensemble avec l'atome d'azote auquel ils sont liés un cycle ayant de 3 à 7 chaînons, en présence d'une base en l'absence ou en la présence d'un solvant entre -10 et 120°C, on met à réagir dans une deuxième étape le 2-chloro-4-dialkylamino-5-fluoronitrobenzène en présence d'une base et d'un catalyseur à base de métaux précieux avec dé l'hydrogène entre 30 et 150°C et entre 1 et 100 bar, on extrait dans une troisième étape du mélange réactionnel la 3-fluoro-4-dialkylaminoaniline avec une solution aqueuse d'un acide sous la forme d'un sel dissous dans l'eau, on sépare la phase aqueuse et on met à réagir le sel de 3-fluoro-4-dialkylaminoaniline dissous dans l'eau en présence d'un composé basique avec un ester d'acide chloro-formique de formule (3) ClCO₂R³, dans laquelle R³ représente un groupe alkyle ayant de 1 à 10 atomes de carbone ou un groupe aralkyle ayant de 7 à 20 atomes de carbone, entre 0 et 100°C.

2. Procédé selon la revendication 1, **caractérisé en ce qu'**on utilise comme amine secondaire de formule (2) la diméthylamine, la diéthylamine, la di-n-propylamine, la di-i-propylamine, la di-n-butylamine, la di-i-butylamine, la pipéridine, la morpholine ou la pipérazine.

3. Procédé selon la revendication 1 ou 2, **caractérisé en ce qu'**on utilise comme base dans la première étape l'amine secondaire de formule (2) ou une amine tertiaire.

4. Procédé selon une ou plusieurs des revendications 1 à 3, **caractérisé en ce qu'**on utilise comme base dans la première étape une trialkylamine, dont les groupes sont identiques ou différents et qui présentent respectivement par groupe alkyle de 1 à 25 atomes de carbone.

5. Procédé selon une ou plusieurs des revendications 1 à 4, **caractérisé en ce qu'**on utilise dans la première étape la base en une quantité de 50 à 500 % en mol, par rapport à l'équivalent en chlore ou en fluor à cliver.

6. Procédé selon une ou plusieurs des revendications 1 à 5, **caractérisé en ce qu'**on utilise comme solvant un hydrocarbure aliphatique ayant de 5 à 25 atomes de carbone, un hydrocarbure aromatique ayant de 6 à 12 atomes de carbone, un alcool aliphatique ayant de 1 à 12 atomes de carbone, un polyalkylèneglycol ayant de 2 à 6 atomes de carbone par groupe alkylène respectif, un éther dialkylique ayant de 2 à 20 atomes de carbone par groupe alkyle, un éther dialkylique de polyalkylèneglycol ayant de 1 à 6 atomes de carbone par groupe alkylène, un dialkylcarboxamide, un acétate d'alkyle en C₁ à C₄, un nitrile, un dialkylsulfoxyde, une dialkylsulfone, une imidazolinone, une pyrrolidone ou un mélange de ceux-ci.

7. Procédé selon une ou plusieurs des revendications 1 à 6, **caractérisé en ce qu'**on utilise comme base dans la deuxième étape l'amine secondaire de formule (2) ou une amine tertiaire.

8. Procédé selon une ou plusieurs des revendications 1 à 7, **caractérisé en ce qu'**on utilise comme base dans la deuxième étape une trialkylamine, dont les groupes sont identiques ou différents et présentent de 1 à 25 atomes de carbone respectivement par groupe alkyle.

9. Procédé selon une ou plusieurs des revendications 1 à 8, **caractérisé en ce qu'**on utilise dans la deuxième étape la base en une quantité de 50 à 500 % en mol, par rapport à l'équivalent en chlore à cliver.

10. Procédé selon une ou plusieurs des revendications 1 à 9, **caractérisé en ce qu'**on utilise comme catalyseur à base de métal précieux un catalyseur-support au palladium.

11. Procédé selon une ou plusieurs des revendications 1 à 10, **caractérisé en ce qu'**on utilise comme acide un acide minéral.

12. Procédé selon une ou plusieurs des revendications 1 à 11, **caractérisé en ce qu'**on utilise comme composé basique un oxyde, un carbonate ou un hydrogénocarbonate d'un métal alcalin ou d'un métal alcalino-terreux ou une amine ou un mélange de ceux-ci.

13. Procédé selon une ou plusieurs des revendications 1 à 12, **caractérisé en ce qu'**on utilise respectivement par mole d'ester d'acide chloro-formique de formule (3) de 0,5 à 3 équivalents de composé basique.

14. Les composés 2-chloro-4-diéthylamino-5-fluoronitrobenzène, 2-chloro-5-fluoro-4-morpholinonitrobenzène, 2-chloro-5-fluoro-4-pipéridinonitrobenzène et 2-chloro-5-fluoro-4-pipérazinonitrobenzène.

15. Les composés 2-chloro-5-fluoro-4-morpholinoaniline et 2-chloro-5-fluoro-4-pipérazinoaniline.
